# EUROPÄISCHE PATENTSCHRIFT

(11) **EP 0 716 605 B1**
(45) Veröffentlichungstag und Bekanntmachung des Hinweises auf die Patenterteilung: **09.12.1998**
(21) Anmeldenummer: 94927587.9
(22) Anmeldetag: 08.09.1994
(51) Int. Cl.: A61K 31/70

(54) **BLOCKIERUNG DER ANLAGERUNG VON KEIMEN AN MENSCHLICHE ZELLEN**
BLOCKING THE ACCUMULATION OF GERMS ON HUMAN CELLS
BLOCAGE DU DEPOT DE GERMES SUR DES CELLULES HUMAINES

(30) Priorität: 10.09.1993 DE 4330773
(43) Veröffentlichungstag der Anmeldung: 19.06.1996
(73) Patentinhaber: Guggenbichler, Josef Peter, Prof. Dr., 90762 Fürth (DE); Jurenitsch, Johann, Prof. Dr., 1020 Wien (AT); de Bettignies-Dutz, Andreas, Dr., 1130 Wien (AT)
(72) Erfinder: GUGGENBICHLER, Josef Peter, Prof. Dr., D-90762 Fürth (DE); MEISSNER, Peter, D-91056 Erlangen (DE); JURENITSCH, Johann, Prof. Dr., A-1020 Wien (AT); DE BETTIGNIES-DUTZ, Andreas, Dr., A-1130 Wien (AT)
(74) Vertreter: Huber, Bernhard, Dipl.-Chem.
(86) Internationale Anmeldenummer: EP9403006
(87) Internationale Veröffentlichungsnummer: WO9507084

(56) Entgegenhaltungen:
- EP-A- 0 080 442
- BIOLOGIA (BRATISLAVA), 1991 Seiten 1081 - 1087 K. HEINRICHOVA, ET AL. 'Preparation of oligo-(d-galactosiduronic) acids by the action of endo-D-galacturonanase'

## Beschreibung

Die vorliegende Erfindung betrifft die Verwendung eines pharmazeutischen Präparats, das als Wirkstoff ein Oligogalakturonid enthält, zur Blockierung der Anlagerung von Keimen an Säugerzellen.

Galakturonsäuren haben ein breites Anwendungsgebiet, wie etwa in Heinrichová und Simon (Biologia, Vol. 46, S. 1081-1087, 1991) beschrieben. Bekannte Anwendungen umfassen beispielsweise die Verwendung zur Kategorisierung und Klassifizierung von Galakturonanasen und Pektatlyasen oder als Liganden für selektive Bioaffinitätschromatographie. Zu den bekannten Wirkungen zählen das Auslösen von Verteidigungsmechanismen höherer Pflanzen und eine Beseitigung toxischer Kationen, wie etwa Cd²⁺, Cu²⁺ und Pb²⁺ im menschlichen Gastrointestinaltrakt.

Wäßrige Auszüge sowie Säfte aus verschiedenen Pflanzenprodukten sind in der Volksmedizin und in der klinischen Medizin für ihre Wirkungen gegen durch pathogene Keime hervorgerufene Erkrankungen im Intestinal- und Urogenitaltrakt bekannt. Die pharmazeutische Wirkung dieser Präparate im Intestinaltrakt wurde bisher mit einer Regulation des Wasserhaushalts durch in den Pflanzenprodukten vorhandene Pektine erklärt.

Die Adhärenz von Keimen, wie etwa bakteriellen Mikroorganismen an Zellen ist der erste Schritt beim Beginn einer jeden Infektion. Erst wenn sich die Keime (z.B. Bakterien oder/und Viren) an spezifische Rezeptoren von Zelloberflächen anheften und dadurch der unspezifischen körpereigenen Abwehr wie Peristaltik, mucilläre Clearance, Sekretfluß etc. entgehen, können sie sich dort vermehren, in den Körper eindringen, toxische Wirkungen entfalten und damit ein Krankheitsbild erzeugen.

Die EP-A- 0 080 442 offenbart ein mehrstufiges chemisches Syntheseverfahren zur Herstellung von Oligosacchariden, wie etwa Oligogalaktosiden aus Pektin bzw. Polygalakturonsäure. Eine pharmazeutische Verwendung der Oligosaccharide und acylierter Derivate davon ist vorgesehen, ausgehend von Berichten, die eine inhibierende Wirkung bestimmter Galaktoside bzw. von Methyl- und p-Nitrophenylderivaten davon, auf die Adhärenz von uropathogenen Coli-Bakterien an bestimmte rote Blutzellen nahelegen.

Ein eleganter Schritt der antimikrobiellen Therapie wäre es daher, die Adhärenz von Keimen als wesentlichen Virulenzmechanismus außer Kraft zu setzen und dadurch dem Körper zu ermöglichen, Keime von Zelloberflächen wegzuschwemmen bzw. von dort zu verdrängen. Die der vorliegenden Erfindung zugrundeliegende Aufgabe bestand somit darin, ein Präparat bereitzustellen, welches die Anlagerung von Keimen an Säugerzellen, insbesondere an Epithelzellen, blockiert.

Diese Aufgabe wird gelöst durch die Verwendung eines pharmazeutischen Präparats, welches dadurch gekennzeichnet ist, daß es als Wirkstoff ein oder mehrere Galakturonide mit einem Polymerisationsgrad ≥ 2 und einem Veresterungsgrad < 20 %, gegebenenfalls zusammen mit pharmazeutisch üblichen Träger-, Verdünnungs-, Hilfs- und Füllstoffen, enthält.

Überraschenderweise wurde gefunden, daß durch Karottensuppe in der klassischen Zubereitung nach MORO, Blasentee (z.B. eine Mischung aus Zinnkraut, gelbem Himmelschlüssel, Erdbeerblättern und Isländischem Moos), Kokosmilch, Kranbeeren etc. die Adhärenz pathogener Keime, wie etwa E.coli, an Zellen, insbesondere an Epithelzellen des Gastrointestinal- und Urogenitaltrakts wesentlich (d.h. bis zu 90 %) reduziert werden kann. Diese Wirkung ist auf die in den Pflanzenprodukten vorhandenen Pektine zurückzuführen, bei denen es sich im wesentlichen aus Ketten von 1,4-α-glycosidisch verbundenen Galakturoniden handelt, deren Säuregruppen zu 20 bis 80 % mit Methanol verestert sind, und die neben Galakturonsäure gegebenenfalls noch andere Zuckerbausteine, z.B. Glucose, Galactose, Xylose und Arabinose enthalten können.

Überraschenderweise wurde festgestellt, daß Pektine bzw. Galakturonide, die bisher zur Bildung von Gelees, als Verdickungsmittel oder als Ballaststoffe verwendet wurden, die Anlagerung von Keimen an Säugerzellen und insbesondere an menschliche Zellen blockieren.

Ein zur Blockierung der Adhärenz von Keimen wirksames Galakturonid muß einen bestimmten Polymerisationsgrad und vorzugsweise einen bestimmten Veresterungsgrad aufweisen. Der Begriff "Polymerisationsgrad" im Sinne der vorliegenden Erfindung bezeichnet die Anzahl der Galakturonsäureinheiten, die ein Galakturonid enthält. Der Begriff "Veresterungsgrad" bezeichnet die Prozent (meist durch Methyl) veresterten Galakturonsäureeinheiten eines Galakturonids bezüglich der Gesamtzahl von Galakturonsäureeinheiten. Es wurde festgestellt, daß zur Blockierung der Adhärenz von Keimen an Säugerzellen ein Polymerisationsgrad ≥ 2 erforderlich ist, d.h. monomere Galakturonsäure zeigt - ebenso wie eine Vielzahl anderer Saccharide (z.B. neutrale Saccharide, Glucuronsäure, Arabinogalaktan, Galaktose-1-phosphat, Glucose-1-phosphat) - keine Blockierung der Adhärenz von Keimen.

In einer bevorzugten Ausführungsform des erfindungsgemäßen pharmazeutischen Präparats ist der Veresterungsgrad der Galakturonide < 20 %, besonders bevorzugt < 10 % und noch mehr bevorzugt < 5 %. Am meisten bevorzugt sind im wesentlichen keine (< 2 %) Estergruppen mehr vorhanden.

Galakturonide mit einem Veresterungsgrad < 20 % sind aus natürlich vorkommenden Pektinen durch vollständige oder teilweise Demethylierung, z.B. durch Verseifung mit Alkali, erhältlich.

Der Polymerisationsgrad von Oligogalakturoniden in einem erfindungsgemäßen pharmazeutischen Präparat ist ≥ 2, d.h. das Galakturonid weist mindestens 2 Galakturonsäureeinheiten auf. Vorzugsweise ist der Polymerisationsgrad des Galakturonids 2 bis 7, noch mehr bevorzugt 2 bis 4, besonders bevorzugt 2 bis 3 und am meisten bevorzugt 2. D.h. Digalakturonid, Trigalakturonid, Tetragalakturonid, Pentagalakturonid, Hexagalakturonid, Heptagalakturonid und Mischungen von mehreren dieser Substanzen sind bevorzugte Galakturonide für die erfindungsgemäßen Präparate, wobei Digalakturonid bzw. Digalakturonsäure am meisten bevorzugt ist. Abhängig von der Art der Anwendung können jedoch auch Präparate verwendet werden, die nicht die von der Wirkung her beste Substanz enthalten, wenn diese Substanz aus pharmazietechnischen Gründen für eine spezielle Anwendungsform weniger günstig ist.

Galakturonide mit einem Polymerisationsgrad von 2 bis 4 sind aus natürlich vorkommenden, höhermolekularen Pektinen durch enzymatische Hydrolyse unter Verwendung von Pektin-spaltenden Enzymen (z.B. Pektinasen) erhältlich, die in der Technik meist aus Pilzen, wie etwa Aspergillus- oder Penicilliumarten gewonnen werden. Andererseits kann die Hydrolyse der höhermolekularen Pektine auch durch saure Hydrolyse z.B. mit HCl erfolgen.

Die erfindungsgemaßen pharmazeutischen Präparate können die Anlagerung von Keimen, insbesondere von bakteriellen Keimen (z.B. E.coli, Streptokokken, Haemophilus influenzae, Pneumokokken etc.), an Säugerzellen, blockieren bzw. bereits gebundene Keime verdrängen. Insbesondere eignen sich die erfindungsgemäßen Präparate zur Behandlung von Infektionen des Gastrointestinaltrakts (z.B. Fehlbesiedelung oberer Dünndarmabschnitte bei Durchfallerkrankungen), des Blutsystems (hämolytisches urämisches Syndrom, durch E.coli O157 verursacht), der Atemwege (z.B. aufsteigende Infektionen bei einem beatmeten Intensivpatienten), des Urogenitaltrakts (z.B. rezividierende Infektionen des Harntrakts) oder/und des Nasen-Rachenraums (z.B. durch Streptokokken, H. influenzae, Pneumokokken).

Die erfindungsgemäßen pharmazeutischen Präparate können einerseits bei einer bereits bestehenden Krankheit und andererseits auch prophylaktisch gegeben werden. Beispielsweise kommen folgende Verabreichungsmöglichkeiten in Betracht:
a) orale Verabreichung etwa zur Behandlung des Gastrointestinaltraktes, Harntraktes, als Zusatz zu Rehydratationslösungen, als Prophylaktikum gegen Fehlbesiedelungen im Magen-Darmtrakt etc., etwa bei Intensivpatienten mit künstlicher Beatmung,
b) topische Anwendungen etwa zur Behandlung des Nasen-Rachenraums, Urogenitaltrakts etc., und
c) parenterale Anwendung.

Galakturonide kommen als Teilstrukturen von Polysacchariden in einer großen Anzahl von Nahrungsmitteln und Lebensmittelhilfsstoffen vor und werden somit peroral eingenommen, d.h. sie sind nicht toxisch und eignen sich hervorragend für pharmazeutische Anwendungen.

Die Gewinnung der für die erfindungsgemäßen pharmazeutischen Präparate verwendeten Galakturonide kann aus in Pflanzen vorkommenden Polysacchariden erfolgen, z.B. aus Karotten, Zitrusfrüchten, Äpfeln, Quitten, Zuckerrüben, Kokosmilch und anderen Pflanzen oder Pflanzenprodukten.

Zur Gewinnung der Galakturonide werden die Ausgangsmaterialien vorzugsweise zerkleinert und mit heißem Wasser extrahiert. Die Extrakte können zuerst lyophilisiert oder auch direkt weiterverarbeitet werden. Die Weiterverarbeitung erfolgt vorzugsweise mit chromatograhischen Methoden, durch die eine Abtrennung der Galakturonide von anderen Bestandteilen des Extrakts (z.B. anderen Sacchariden) möglich ist. Bevorzugte chromatographische Trennmethoden sind Gelchromatographie (z.B. BioGel®-, Sephacryl®-Trennmittel) oder/und Anionenaustauscherchromatographie (z.B. DEAE-Sephacel®-Trennmaterialien). Die erhaltenen Pektine können weiterhin zur Verringerung des Veresterungsgrades teilweise oder vollständig verseift oder/und zur Verringerung des Polymerisationsgrades durch Säurebehandlung bzw. enzymatisch hydrolysiert werden.

Die Erfindung soll weiterhin durch die vorliegenden Beispiele erläutert werden.

### Beispiel 1:

Frische Karotten werden gewaschen, geschält und geraspelt. Durch Extraktion mit siedendem Wasser und darauffolgende Lyophilisation werden die wasserlöslichen Polysaccharide gewonnen.

Es folgt eine erste gelchromatographische Trennstufe über eine Säule mit BioGel P2 und destilliertem Wasser als Eluent. Die Substanzen, die am Ausschlußvolumen dieser Säule eluieren (SF A) werden vereinigt und ein zweites Mal gelchromatographisch aufgetrennt. Als Trennmaterial dient Sephacryl S-3000HR und als Eluent nochmals destilliertes Wasser. Von den zwei Substanzpeaks, die nun erscheinen, wird der später eluierende gesammelt.

Der dritte Aufbereitungsschritt ist eine Ionenaustauscherchromatographie über DEAE-Sephacel mit einem Puffergradienten als Eluent (0,011 M bis 1 M Phosphatpuffer, pH 6,4). Die Fraktion, die mit höherer Molarität eluiert wird (SF II), wird gepoolt und weiterverarbeitet. Daraufhin erfolgt eine Hydrolyse mit HCl (pH 1,00, 37°C, 45 min) und die Wiedergewinnung der Saccharide durch Alkoholfällung.

Das Hydrolysat wird auf gelchromatographischem Weg (Sephacryl S-2000HR, Eluent: destilliertes Wasser) in drei Substanzgruppen aufgetrennt, wobei die Kohlenhydrate mit dem geringsten Molekulargewicht (SF 2/3) gesammelt werden.

Die antiadhäsive Aktivität der Fraktionen, mit denen weitergearbeitet wird, übertrifft die der jeweils anfallenden Nebenprodukte.

Die Hemmintensität der einzelnen Substanzen steigt im Laufe der Aufarbeitung von 50 % in 0,7 %iger Lösung (bei SF A) über 86,4 % in 0,005 %iger Lösung (bei SF II) auf vollständige Hemmung ebenfalls in 0,005 %iger Lösung (bei SF 2/3).

Dies deutet darauf hin, daß mit zunehmender Reinheit die zur Erzielung einer bestimmten Wirkung notwendige Konzentration deutlich geringer wird. Dies steht im Gegensatz zu im Handel befindlichen Pektinen, die in sehr hohen Konzentrationen eingesetzt werden müssen, um ähnliche Effekte erzielen zu können.

### Beispiel 2:

### Verseifung:

11,3 g Zitruspektin, Fa. Grindstedt (Substanz A₀) werden in 1200 ml dest. Wasser gelöst, mit 480 ml 0,5 N NaOH versetzt und 15 min bei Raumtemperatur verseift. Die Lösung wird mit Ameisensäure konz. auf pH 4,0 eingestellt. Das verseifte Pektin wird mit dem doppelten Volumen Methanol gefällt, in 200 ml Wasser gelöst und die Fällung noch zwei Mal in gleicher Weise wiederholt. Der zuletzt gewonnene Niederschlag wird bei 50°C getrocknet (Substanz A₁ ). Auswaage: 7,15 g.

### Enzymatische Hydrolyse:

Der getrocknete Niederschlag wird mit 100 ml 0,1 M Natriumhydrogencarbonat-Lösung gelöst, mit Ameisensäure (1 M) auf pH 4,5 eingestellt, mit 21 mg Pectinase 5S aus Aspergillus niger (Fa. Serva) versetzt und 60 min bei 60°C inkubiert. Der Ansatz wird zur Enzymaktivierung kurze Zeit auf 80°C erwärmt, abgekühlt und in oben beschriebener Weise mit Methanol gefällt. Durch Zentrifugieren (20 Minuten, 4000 U/min) wird der Niederschlag gewonnen und getrocknet. Auswaage: 5 g (Substanz A₂).

Durch dünnschichtchromatographische Kontrolle wird überprüft, ob die Hydrolyse ausreichende Mengen an den gewünschten Oligogalakturoniden erzeugte.

### Dünnschichtchromatographiebedingungen:

- Stationäre Phase:: DC-Fertigplatten Kieselgel 60 (Fa. Merck) 10 cm x 20 cm
- Fließmittel:: Ethanol: wäßrige Essigsäure 25 mM 1:1
- Entwicklung:: bei 35°C
- Sprühreagenz:: 200 mg Naphthalin-1,3-diol in 50 ml Methanol plus 50 ml H₂SO₄ (20 % g/g)

### Chromatographie:

1,5 g des Hydrolyseproduktes werden in 15 ml dest. Wasser gelöst und der chromatographischen Fraktionierung unterworfen.
- Säule:: Glassäule der Fa. Bio-Rad (2,5/40 cm) Bettvolumen 150 ml
- Eluent:: Na-Formiatpuffer pH 4,7, Stufengradient je 182 ml 0,2, 0,3, 0,4, 0,5, 0,6, 0,7 M
- Stationäre Phase:: Bio-Rad AGMP 1 Anionenaustauscherharz (mit Eluent äquilibriert)
- Fließrate:: 3 ml/min
- Fraktionsvol.:: 22 ml

Die einzelnen Fraktionen werden mittels DC (Bedingungen siehe oben) auf ihre Zusammensetzung untersucht. Fraktionen gleicher Zusammensetzung werden vereinigt und mit dem zweifachen Volumen Aceton gefällt. Die auf diese Art und Weise gewonnenen Oligogalakturonide werden durch Zentrifugation (4000 U/min) gewonnen und in 20 ml dest. Wasser aufgenommen.

In die Lösung wird Bio-Rad AG 50 W X-8 Kationenaustauscher (H+) eingerührt, um die Galakturonide in die freie Säureform überzuführen. Nach Entfernen des Harzes und Lyophilisieren des Filtrats erhält man flockiges, weißes Pulver.

Die Ergebnisse der Hemmung sind in der folgenden Tabelle 1 dargestellt.

**Tabelle 1**

| Substanzbezeichnung | Charakterisierung | Konz. in % | Blockierung in % |
|---|---|---|---|
| A₀ | Pektin USP¹⁾ | 0,7 | 15,3 |
| A₁ | verseiftes Pektin | 0,2 | 10,0 |
| A₂ | partiell hydrolysiertes A₁ | 0,05 | 30,2 |
| F₁ | neutrales Di- und Trisaccharid | 0,005 | 0,0 |
| F₂ | neutrales Trisaccharid | 0,005 | 0,0 |
| F₃ | neutrales Tetrasaccharid | 0,005 | 0,0 |
| F₄ | Trigalakturonid | 0,005 | 84,6 |
| F₅ | Tetragalakturonid mit Spuren Trigalakturonid | 0,005 | 58,6 |
| F₆ | Tetragalakutronid | 0,005 | 52,7 |
| F₇ | Penta-Hexagalakturonid | 0,005 | 23,9 |

| | | | |
|---|---|---|---|
| ¹⁾ Zitruspektin (Fa. Grindstedt) | | | |

### Methoden zur Prüfung der Hemmungswirksamkeit:

### Adhäsionstests:

1) Hämagglutination vom menschlichen O+ Erythrozyten: Nach Zusammenbringen von Erythrozyten mit Keimen kommt es bei stark hämagglutinierenden Stämmen innerhalb von 20 Sekunden zu einer Agglutination von Erythrozyten, die der Stärke einer Blutgruppenunverträglichkeit entspricht.
   Konzentration von Erythrozyten: 1 - 1,5 %
   Keimsuspension: trübe Suspension OD 1 bei 462 nm = 10⁹ Keime/ml
   Bei der Bewertung der Agglutinationsblockierung werden die Keime vor Zugabe zu den Erythrozyten für 2 h mit den Kohlenhydratlösungen in der jeweils gewünschten Konzentration inkubiert und dann die Hämagglutination mit dem Auge semiquantitativ bewertet.
2) Adhärenz an menschlichen Uroepithelien: Uroepithelien aus dem Morgenharn werden durch Zentrifugation isoliert. Keimsuspensionen (wie oben) werden mit Epithelzellen zusammengebracht und inkubiert, danach durch 8 µ Polycarbonatfilter filtriert und mehrfach gewaschen. Filter werden entfernt, in physiologische NaCl-Lösung eingebracht und die Epithelzellen abgeschüttelt. Die NaCl-Suspension wird nun wiederum zentrifugiert und das Sediment auf Objektträger aufgebracht, nach May Grünwald und Giemsa gefärbt und die haftenden Keime an 50 Epithelzellen gezählt (Leerwert).
   Kontrollen: Epithelzellen ohne Keimkontakt.
   Adhärenzblockierung: Gleiche Methode, nur werden die Keime vor Zugabe zur Epithelzellensuspension für 1 - 3 Stunden in eine Lösung (0,5, 0,1, 0,05, 0,001 %) der verschiedenen Kohlenhydrate eingebracht.
   Verwendete Keime: 4 E.coli-Wildtypstämme, die von Patienten isoliert wurden, und 2 käufliche Stämme werden gleichermaßen eingesetzt.

### Ergebnisse:

Im Rahmen der mikrobiologischen Untersuchungen wurden neben unten taxativ aufgezählten sauren Oligo- und Polysacchariden auch neutrale Saccharide eingesetzt, die keinerlei adhärenzblockierende Wirkung zeigten (z.B. Fructane wie Inulin, Disaccharide wie Lactose und Lactulose u.a.m.).

Native, d.h. durch Extraktion gewonnene Pektine zeigen eine Adhärenz blockierende Wirkung, welche jedoch durch weitere Behandlung, z.B. Verseifung und Hydrolyse, gesteigert werden konnte.

So zeigte im oben beschriebenen Testansatz das durch wäßrige Extraktion aus Karotten gewonnene Pektin in 1 %iger Lösung im Testansatz eine 46 %ige Hemmung, die aufgereinigten Produkte im selben Ansatz in einer Konzentration von 0,1 % 80 %ige Hemmung.

In der folgenden Tabelle 2 sind weitere Ergebnisse der mikrobiologischen Untersuchungen zusammengefaßt:

Blockierung der bakteriellen Adhäsion an Epithelzellen durch ausgewählte Substanzen in %:

**Tabelle 2**

| Substanzbezeichnung | Konzentration in % | Blockierung in % |
|---|---|---|
| Karottenwasserextrakt¹⁾ | 1 | 46,0 |
| SF A (s. Bsp. 1) | 0,7 | 50,0 |
| SF II (s. Bsp. 1) | 0,005 | 86,4 |
| SF 2/3 (s. Bsp. 1) | 0,005 | 113,5 |
| Polygalakturonsäure aus Orangen | 0,2 | 99,0 |
| Polygalakturonsäure aus Orangen | 0,05 | 72,0 |
| Monogalakturonsäure | 1 | keine Hemmung |
| Digalakturonsäure | 0,005 | 91,7 |
| Trigalakturonid²⁾ | 0,005 | 84,6 |
| Tetragalakturonid²⁾ | 0,005 | 52,7 |
| Penta-Hexagalakturonid | 0,005 | 23,9 |
| Arabinogalaktan | 0,005 | keine Hemmung |
| Apfelpektin | 0,05 | 20,5 |
| Pektinsäure aus Apfel | 0,05 | 64,8 |
| Galaktose-1-Phosphat | 0,005 | 3,4 |
| Glucose-1-Phosphat | 0,005 | keine Hemmung |
| Glucuronsäure | 0,005 | keine Hemmung |

| | | |
|---|---|---|
| ¹⁾ Analog Beispiel 1 | | |
| ²⁾ Gewonnen analog Beispiel 2 | | |

Wie aus Tabelle 2 ersichtlich, läßt sich eine Wirkungszunahme in Abhängigkeit vom Veresterungsgrad und der Molekülgröße (dem Polymerisationsgrad) ableiten.

Günstige Ergebnisse werden bei einem Polymerisationsgrad von 2 - 7 erhalten.

Durch eine Verseifung (d.h. Entfernung von Estergruppen) wird ebenfalls eine Verbesserung der Blockierung gefunden.

## Patentansprüche

1. Verfahren zur Herstellung eines pharmazeutischen Präparats zur Blockierung der Anlagerung von Keimen an Säugerzellen,
**dadurch gekennzeichnet,**
daß man ein oder mehrere Oligogalakturonide mit einem Polymerisationsgrad ≥ 2 und einem Veresterungsgrad < 20 % als Wirkstoff, gegebenenfalls zusammen mit pharmazeutisch üblichen Träger-, Verdünnungs-, Hilfs- und Füllstoffen in eine zur Verabreichung geeignete Form bringt.

2. Verfahren nach Anspruch 1,
**dadurch gekennzeichnet,**
daß der Veresterungsgrad des Galakturonids < 10 % ist.

3. Verfahren nach Anspruch 1 oder 2,
**dadurch gekennzeichnet,**
daß der Veresterungsgrad des Galakturonids < 5 % ist.

4. Verfahren nach einem der Ansprüche 1 bis 3,
**dadurch gekennzeichnet,**
daß der Polymerisationsgrad des Galakturonids 2 bis 7 ist.

5. Verfahren nach einem der Ansprüche 1 bis 4,
**dadurch gekennzeichnet,**
daß der Polymerisationsgrad des Galakturonids 2 bis 6 ist.

6. Verfahren nach einem der Ansprüche 1 bis 5,
**dadurch gekennzeichnet,**
daß der Polymerisationsgrad des Galakturonids 2 bis 5 ist.

7. Verfahren nach einem der Ansprüche 1 bis 6,
**dadurch gekennzeichnet,**
daß der Polymerisationsgrad des Galakturonids 2 bis 4 ist.

8. Verfahren nach einem der Ansprüche 1 bis 7,
**dadurch gekennzeichnet,**
daß der Polymerisationsgrad des Galakturonids 2 bis 3 ist.

9. Verfahren nach einem der Ansprüche 1 bis 8,
**dadurch gekennzeichnet,**
daß der Polymerisationsgrad des Galakturonids 2 ist.

10. Verfahren nach einem der Ansprüche 1 bis 9 für die Herstellung eines pharmazeutischen Präparats zur Behandlung von Infektionen des Gastrointestinaltrakts, des Blutsystems, der Atemwege, des Urogenitaltrakts oder/und des Nasen-Rachenraums.

## Claims

1. Process for the production of a pharmaceutical preparation to block the attachment of germs to mammalian cells
**wherein**
one or several oligogalacturonides as the active substance with a degree of polymerization of ≥ 2 and a degree of esterification of < 20 %, optionally together with common pharmaceutical carriers, diluents, auxiliary substances and fillers are made into a form suitable for administration.

2. Process as claimed in claim 1,
**wherein**
the degree of esterification of the galacturonide is < 10 %.

3. Process as claimed in claim 1 or 2,
**wherein**
the degree of esterification of the galacturonide is < 5 %.

4. Process as claimed in one of the claims 1 to 3,
**wherein**
the degree of polymerization of the galacturonide is 2 to 7.

5. Process as claimed in one of the claims 1 to 4,
**wherein**
the degree of polymerization of the galacturonide is 2 to 6.

6. Process as claimed in one of the claims 1 to 5,
**wherein**
the degree of polymerization of the galacturonide is 2 to 5.

7. Process as claimed in one of the claims 1 to 6,
**wherein**
the degree of polymerization of the galacturonide is 2 to 4.

8. Process as claimed in one of the claims 1 to 7,
**wherein**
the degree of polymerization of the galacturonide is 2 to 3.

9. Process as claimed in one of the claims 1 to 8,
**wherein**
the degree of polymerization of the galacturonide is 2.

10. Process as claimed in one of the claims 1 to 9 for the production of a pharmaceutical preparation to treat infections of the gastrointestinal tract, the blood system, the respiratory passages, the urogenital tract or/and the nasopharyngeal space.

## Revendications

1. Procédé pour la fabrication d'une préparation pharmaceutique destinée au blocage de dépôt de germes sur des cellules humaines,
caractérisé en ce que
l'on met sous une forme appropriée à l'administration un ou plusieurs oligogalacturonides avec un degré de polymérisation supérieur ou égal à 2 et un degré d'estérification inférieur à 20 % en tant que principe actif, éventuellement avec les supports, adjuvants, matières de charge et de dilution usuelles.

2. Procédé selon la revendication 1,
caractérisé en ce que
le degré d'estérification de l'oligogalacturonide est inférieur à 10 %.

3. Procédé selon la revendication 1 ou 2,
caractérisé en ce que
le degré d'estérification de l'oligogalacturonide est inférieur à 5 %.

4. Procédé selon l'une des revendications 1 à 3,
caractérisé en ce que
le degré de polymérisation de l'oligogalacturonide est de 2 à 7.

5. Procédé selon l'une des revendications 1 à 4,
caractérisé en ce que
le degré de polymérisation de l'oligogalacturonide est de 2 à 6.

6. Procédé selon l'une des revendications 1 à 5,
caractérisé en ce que
le degré de polymérisation de l'oligogalacturonide est de 2 à 5.

7. Procédé selon l'une des revendications 1 à 6,
caractérisé en ce que
le degré de polymérisation de l'oligogalacturonide est de 2 à 4.

8. Procédé selon l'une des revendications 1 à 7,
caractérisé en ce que
le degré de polymérisation de l'oligogalacturonide est de 2 à 3.

9. Procédé selon l'une des revendications 1 à 8,
caractérisé en ce que
le degré de polymérisation de l'oligogalacturonide est de 2.

10. Procédé selon l'une des revendications 1 à 9, pour la fabrication d'une préparation pharmaceutique destinée au traitement d'infections des voies gastro-intestinales, du système sanguin, des voies respiratoires, des voies urinogénitales et/ou de l'espace nasopharyngien.
